# EUROPEAN PATENT SPECIFICATION

(11) **EP 3 903 735 B1**
(45) Date of publication and mention of the grant of the patent: **19.04.2023**
(21) Application number: 19904549.3
(22) Date of filing: 24.12.2019
(51) Int. Cl.: A61F 2/24, A61F 2/95

(54) **LOADING TOOL FOR IMPLANT AND MEDICAL DEVICE**
LADEWERKZEUG FÜR IMPLANTAT UND MEDIZINISCHE VORRICHTUNG
OUTIL DE CHARGEMENT POUR IMPLANT ET DISPOSITIF MÉDICAL

(30) Priority: 28.12.2018 CN 201811653500
(43) Date of publication of application: 03.11.2021
(73) Proprietor: Shanghai MicroPort CardioFlow Medtech Co., Ltd., Shanghai 201203 (CN)
(72) Inventor: MEI, Jie, Shanghai 200135 (CN); LIU, Meichen, Shanghai 200135 (CN); WU, Xuwen, Shanghai 200135 (CN); GUI, Baozhu, Shanghai 200135 (CN); CHEN, Guoming, Shanghai 200135 (CN); LI, Yu, Shanghai 200135 (CN)
(74) Representative: Lavoix
(86) International application number: PCT/CN2019/127986
(87) International publication number: WO 2020/135430

(56) References cited:
- EP-A1- 3 064 174
- US-A1- 2015 081 011

## Description

### TECHNICAL FIELD

The present invention relates to the field of medical instruments and, in particular, to a loading tool for implant and a medical device.

### BACKGROUND

Interventional aortic valve implantation is a novel minimally invasive valve replacement technique that has been developed abroad in recent years, which involves loading a valve prosthesis (including a stent) in a delivery device and delivering it through a catheter of the delivery device to the aortic root, where the stent is released to ensure that the valve prosthesis is anchored to the aortic valve annulus, thus functionally replacing the dysfunctional native valve and improving the patient's cardiac condition. This technique is able to treat aortic valve disease without surgically opening the chest or stopping the heartbeat, which may cause significant trauma to the patient.

European patent application No. EP3064174A1 discloses a device and method for loading an implant into a delivery system. The device includes a guide cap, a guider and a guiding tube. The guide cap has a conical section and a straight or conical tube in communication with a small open end of the conical section, the conical section having a large open end that flares outward and thereby forms a flange. The guider has a circumferential wall tapered along an axis of the guider and thus forms a large open end at a first edge and a small open end at a second edge of the circumferential wall, the small open end of the guider having a diameter greater than a diameter of the small open end of the conical section of the guide cap. The guide cap and the guider are so sized that the flange is able to cover the large open end of the guider, with an end portion of the straight or conical tube protruding from the small open end of the guider. The guiding tube is a round tube with a first and a second open end, the first open end having a diameter greater than the diameter of the small open end of the conical section of the guide cap and smaller than the diameter of the small open end of the guider.

US patent application No. 2015/081011A1 discloses systems and methods for loading a valve prosthesis onto a catheter. The system comprises a first housing, a second housing, and a plate. The first housing comprises a first open end, a first tapered inner surface, and a second open end. The second housing comprises a third open end, a second tapered inner surface, and a fourth open end. The second housing can define a slot between the second tapered surface and the third open end. The plate can be configured to be slidably received within the slot. The loading system can further comprise a first elongated member and a second elongated member.

### SUMMARY OF THE INVENTION

### Technical Problem

This technique requires the stent to be compressed to a diameter that is small enough to allow it to be loaded in the catheter of the delivery device. However, the stent or the valve thereon is prone to damage or breakage due to excessive or uneven compression or accidental local flexing, which may impair the function or shorten a service life of the stent or the valve, or even make the implantation or normal operation thereof impossible. The anchoring and compression of a self-expanding stent is particularly challenging because it will undergo tension when so handled, making the stent more prone to damage or breakage during loading. This on the one hand imposes strict requirements on the operator and, on the other hand, leads to a prolonged surgical procedure and a higher surgical risk.

Loading a valve prosthesis using a loading tool typically involves initially compressing of a stent in the prosthesis by engaging a guide cover with a guide base and subsequently compressing, after the guide cover is disengaged from the guide base, an inflow section of the stent is further compressed by an inner lumen of the guide base until the valve prosthesis is fully crimped. The initial compression is important because it accomplishes compression and partial loading of the stent. However, initial compression using conventional loading tools tends to encounter stent twisting or slanting thereby resulting in deformation or damage of the stent, which may directly exert adverse effects on the subsequent operations, or even lead to failure of loading or impair the subsequent use of the implant.

Therefore, there is a need for easy-to-use, efficient loading device.

### Solution to Problem

It is an object of the present invention to provide a loading tool and a medical device for an implant, which solve the above problems of easy implant deformation or damage due to twisting or slanting arising from the use of conventional loading tools.

To this end, the present invention provides a loading tool for implant according to claim 1.

Further embodiments of the loading tool are described in claims 2 to 13.

The above object is also attained by a medical device according to claim 14 or claim 15.

In summary, in the provided loading tool for an implant and medical device, one of the guide cover and the guide base is provided with the first snap and the second snap, and the other of the guide cover and the guide base is provided with the first snap receptacle and the second snap receptacle. The first snap is configured to form a snap-in fit with the first snap receptacle which to limit radial and axial displacement of the guide cover with respect to the guide base. The second snap is configured to form a snap-in fit with the second snap receptacle to limit radial and axial displacement and circumferential rotation of the guide cover with respect to the guide base. The snap-in fit between the first snap and the first snap receptacle prevents radial displacement between the guide cover and the guide base and limits their relative axial displacement so that they are in a stable coaxial configuration. This facilitates the introduction of a conical tip of a delivery device and thus subsequent compression of an implant. The snap-in fit between the second snap and the second snap receptacle prevents radial displacement, axial displacement and circumferential rotation between the guide cover and the guide base. Immobilizing the guide cover with respect to the guide base can prevent twisting, slanting or abrasion of the implant during compression thereof. The snap-in fits between the snaps and the respective snap receptacles allow easy engagement of the guide cover with the guide base and more efficient loading of the implant.

### BRIEF DESCRIPTION OF THE DRAWINGS

As would be appreciated by those of ordinary skill in the art, the following accompanying drawings are provided to enhance understanding of the present invention and do not limit the scope thereof in any sense, in which:
Fig. 1 is a front view of a guide cover of a loading tool for implant according to an embodiment of the present invention;
Fig. 2 is an axial cross-sectional view of the guide cover of Fig. 1 taken along line A-A;
Fig. 3 is an axial cross-sectional view of a guide cover of a loading tool for implant according to a preferred embodiment of the present invention;
Fig. 4 is a front view of a guide base of a loading tool for implant according to an embodiment of the present invention;
Fig. 5 is an axial cross-sectional view of the guide cover of Fig. 4 taken along line B-B;
Fig. 6 is a front view of a guide cover of a loading tool for implant in engagement with a guide base thereof according to an embodiment of the present invention;
Fig. 7 is an axial cross-sectional view of the loading tool for implant of Fig. 6 taken along line C-C;
Fig. 8 is a schematic illustration of a delivery device according to an embodiment of the present invention;
Fig. 9 is a front view of a delivery device and a loading tool for implant in engagement therewith according to an embodiment of the present invention, in which the delivery device is engaged with a guide base at a fourth end thereof;
Fig. 10is an axial cross-sectional view of the delivery device and the loading tool for implant of Fig. 9, which are in engagement with each other;
Fig. 11 is afront view of a delivery device and a loading tool for implant in engagement therewith according to an embodiment of the present invention, in which the delivery device is engaged with a guide base at a third end thereof;
Fig. 12 is an axial cross-sectional view of the delivery device and the loading tool for implant of Fig. 11, which are in engagement with each other; and
Fig. 13 schematically illustrates an implant according to an embodiment of the present invention.

In these figures,
1: a guide cover; 100: a first inner lumen; 101: a first end; 102: a second end; 11: an indicator ring; 12: a second snap; 121: a first bevel; 122: a third snap surface; 123: a third stop surface; 124: a fifth stop surface; 13: a first section; 14: a fourth section; 15: a third section; 16: a second section; 17: a first snap; 171: a first snap surface; 172: a first engagement surface; 180: a slot; 181, an inter-slot portion; 19: an anti-slip feature;
2: a guide base; 200: a second inner lumen; 201: a third end; 202: a fourth end; 21: a leading end of the guide base; 22: a first snap receptacle; 221: a second snap surface; 222: a second engagement surface; 23: an anchoring trench; 24: a second snap receptacle; 241: a second bevel; 242: a fourth snap surface; 243: a fourth stop surface; 244: a pocket; 25: a sixth section; 26: a fifth section;
5: a delivery device; 54: a conical tip; 55: an anchor; 56: a sheath;
9: a valve stent; 91: an attachment projection; 92: an outflow section; and 93: an inflow section.

### DETAILED DESCRIPTION

The above and other objects, advantages and features of the present invention will become more apparent from the following detailed description of several specific embodiments thereof, which is to be read in conjunction with the accompanying drawings. It is noted that the figures are presented in a very simplified form not necessarily drawn to exact scale for the only purpose of helping to explain the disclosed embodiments in a more convenient and clearer way. In addition, structures shown in the figures are usually portions of actual structures. In particular, as the figures tend to have distinct emphases, they are often drawn to different scales.

As used herein and in the appended claims, the singular forms "a", "an", and "the" include plural references unless the context clearly dictates otherwise. As used herein and in the appended claims, the term "or" is generally employed in its sense including "and/or" unless the content clearly dictates otherwise. The term "proximal" generally refers to the end of an object closer to a physician, and the term "distal" generally refers to the end closer to a lesion.

The present invention provides a loading tool for implant and a medical device, which includes a guide cover and a guide base detachably coupled to the guide cover. The guide cover has a first end and a second end opposing the first end along an axial direction of the guide cover. The guide base has a third end and a fourth end opposing the third end along an axial direction of the guide base. The guide cover has a first inner lumen extending therethrough, and the guide base is configured for insertion into the first inner lumen from the first end toward the second end in the direction from the third end toward the fourth end. One of the guide cover and the guide base is provided with a first snap and a second snap, and the other one is provided with a first snap receptacle and a second snap receptacle. The first snap is configured for a snap-in fit with the first snap receptacle, which limits radial and axial displacement of the guide cover relative to the guide base. The second snap is configured for a snap-in fit with the second snap receptacle, which limits radial and axial displacement and circumferential rotation of the guide cover relative to the guide base. During the insertion of the guide base into the first inner lumen, the distance between the first snap and the first snap receptacle is smaller than that between the second snap and the second snap receptacle. With this arrangement, the snap-in fit between the first snap and the first snap receptacle precedes the snap-in fit between the second snap and the second snap receptacle, and the latter occurs also after the first snap has disengaged from the first snap receptacle. The snap-in fit between the first snap and the first snap receptacle prevents radial displacement between the guide cover and the guide base and limits their relative axial displacement so that they are in a stable coaxial configuration. This facilitates the introduction of a conical tip of a delivery device and thus subsequent compression of the implant. The snap-in fit between the second snap and the second snap receptacle prevents radial displacement, axial displacement and circumferential rotation between the guide cover and the guide base. Fixing the guide cover with respect to the guide base can prevent twisting, slanting or abrasion of the implant during compression thereof. The snap-in fits between the snaps and the respective snap receptacles allow easy engagement of the guide cover with the guide base and more efficient loading of the implant.

A more detailed description is set forth below with reference to the accompanying drawings.

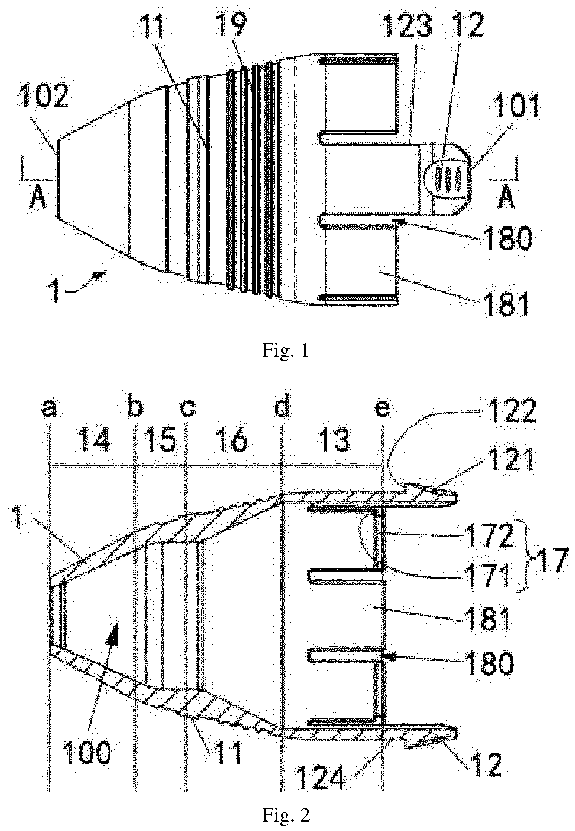

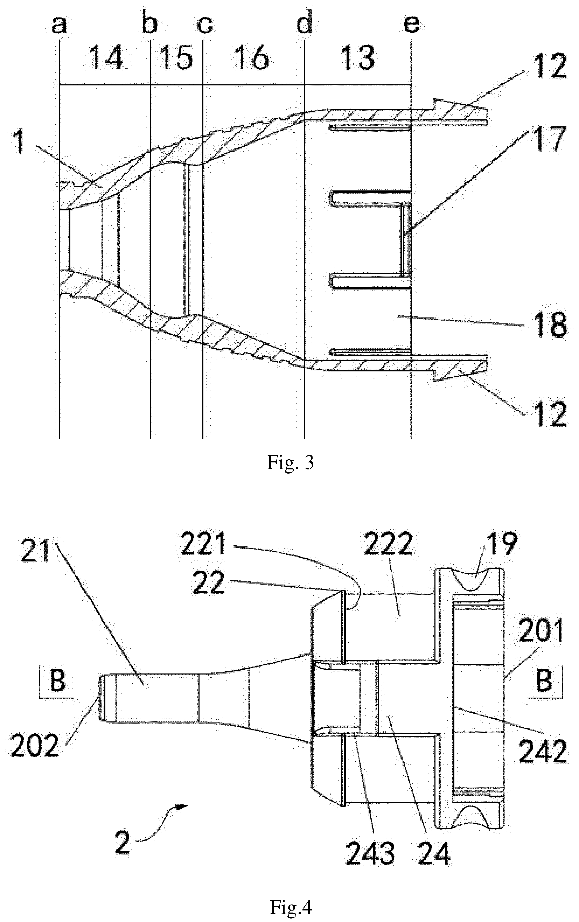

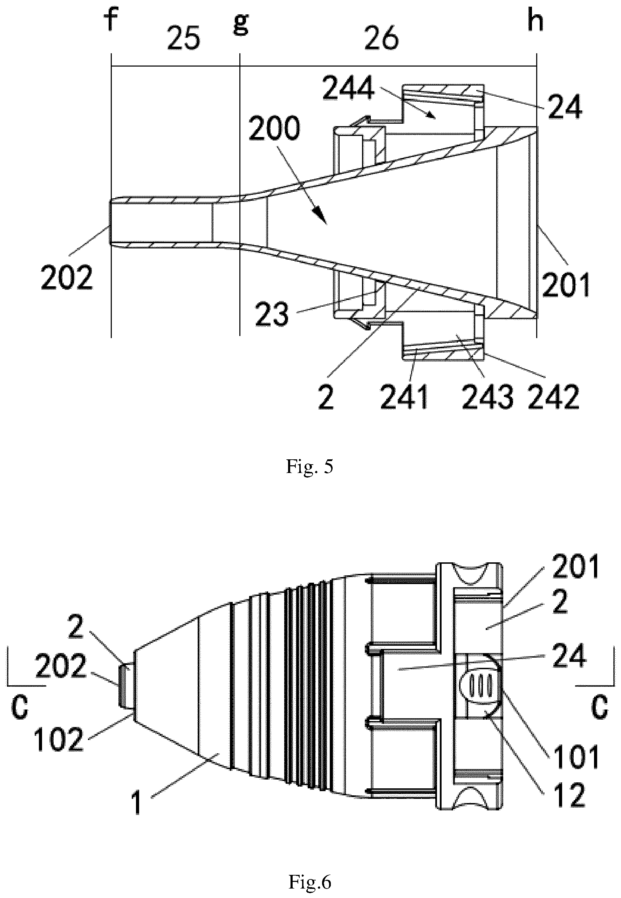

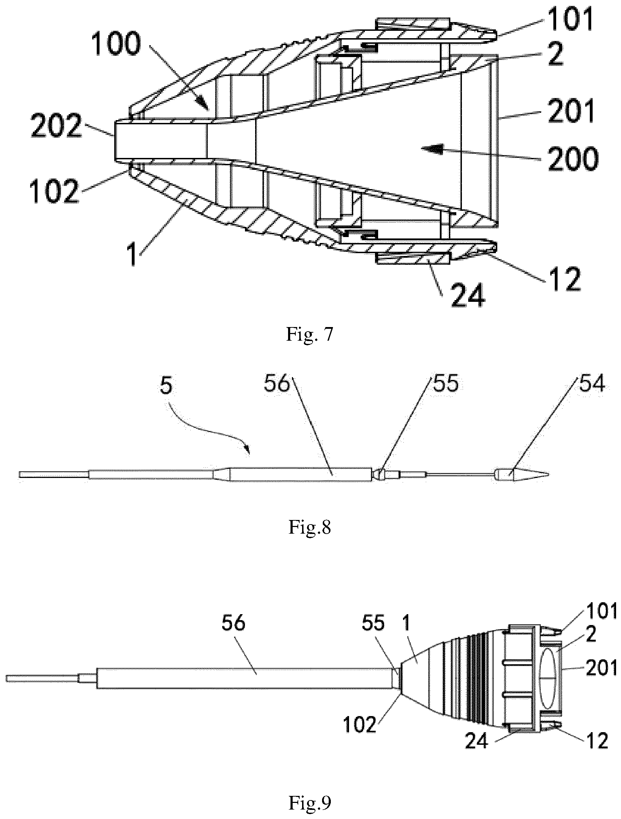

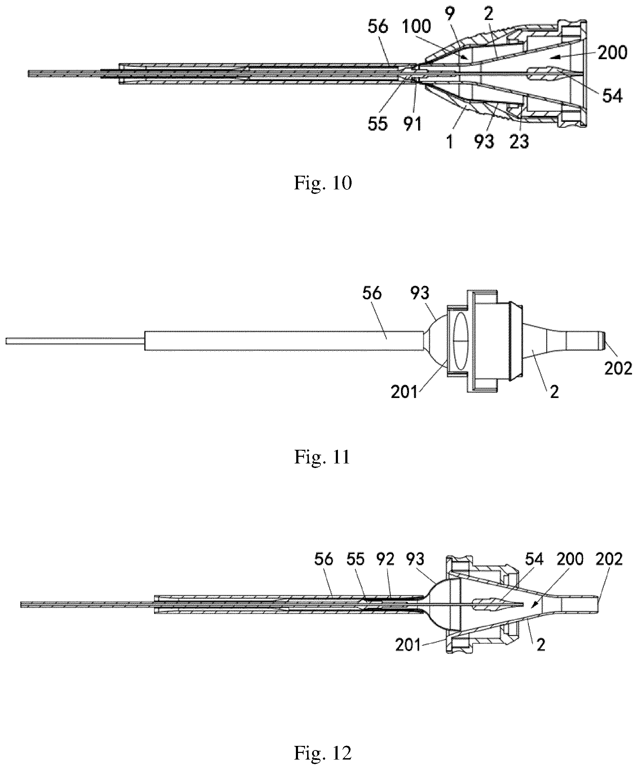

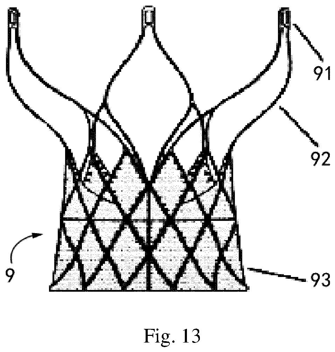

In the following, reference is made to Figs. 1 to 13. Fig. 1 is a front view of a guide cover of a loading tool for implant according to an embodiment of the present invention. Fig. 2 is an axial cross-sectional view of the guide cover of Fig. 1 taken along line A-A. Fig. 3 is an axial cross-sectional view of a guide cover of a loading tool for implant according to a preferred embodiment of the present invention. Fig. 4 is a front view of a guide base of a loading tool for implant according to an embodiment of the present invention. Fig. 5 is an axial cross-sectional view of the guide cover of Fig. 4 taken along line B-B. Fig. 6 is a front view of a guide cover of a loading tool for implant in engagement with a guide base thereof according to an embodiment of the present invention. Fig. 7 is an axial cross-sectional view of the guide cover of Fig. 6 taken along line C-C. Fig. 8 is a schematic illustration of a delivery device according to an embodiment of the present invention. Fig. 9 is afront view of a delivery device and an loading tool for implant in engagement therewith according to an embodiment of the present invention, in which the delivery device is engaged with a fourth end of a guide base at. Fig. 10 is an axial cross-sectional view of the delivery device and the loading tool for implant of Fig. 9, which are in engagement with each other. Fig. 11 is afront view of a delivery device and an loading tool for implant in engagement therewith according to an embodiment of the present invention, in which the delivery device is engaged with a third end of a guide base. Fig. 12 is an axial cross-sectional view of the delivery device and the loading tool for implant of Fig. 11, which are in engagement with each other. Fig. 13 schematically illustrates an implant according to an embodiment of the present invention.

As shown in Figs. 1 to 7, in the present embodiments, there is provided a loading tool for implant including a guide cover 1 and a guide base 2 detachably coupled to the guide cover 1. The guide cover 1 has a first inner lumen 100 extending therethrough, and the guide base 2 is configured to be inserted into the first inner lumen 100 to form an assembly in which an implant is compressed. The guide cover 1 has a first end 101 and a second end 102 opposing the first end 101 along an axial direction of the guide cover 1, and the guide base 2 has a third end 201 and a fourth end 202 opposing the third end 201 along an axial direction. The guide base 2 is configured to be inserted, preferably, coaxially, into the first inner lumen 100 from the first end 101 toward the second end 102 in the direction from the third end 201 toward the fourth end 202.

Preferably, as shown in Figs. 1 and 2, the first inner lumen 100 of the guide cover 1 includes a first section 13, a second section 16, a third section 15 and a fourth section 14, which are sequentially joined from the first end 101 toward the second end 102. The first section 13 has an inner diameter that is maintained constant along the axial direction of the guide cover 1 (i.e., an inner diameter at the cross-section e is equal to that at the cross-section d in Fig. 2). A plurality of slots 180 are formed circumferentially in an end portion of the first section 13 close to the first end 101 and each adjacent two of the slots define an inter-slot portion 181therebetween. In practice, the first section 13 is configured for contact, abutment or engagement with the guide base 2.

The guide cover 1 is provided with a first snap 17 and a second snap 12, and the guide base 2 is provided with a corresponding first snap receptacle 22 and a corresponding second snap receptacle24. The first snap 17 is configured to form a snap-in fit with the first snap receptacle 22, which limits radial and axial displacement of the guide cover 1 with respect to the guide base 2. The second snap 12 is configured for a snap-in fit with the second snap receptacle 24, which limits radial and axial displacement and circumferential rotation of the guide cover 1 with respect to the guide base 2. During the insertion of the guide base 2 into the first inner lumen 100, the distance between the first snap 17 and the first snap receptacle 22 is smaller than the distance between the second snap 12 and the second snap receptacle 24. Here, it is to be noted that the distances between the snaps and the respective snap receptacles refer to their distances along the axial directions of the guide cover 1 and the guide base 2. In addition, each of the distances may be directional. In particular, since the guide base 2 is configured to be inserted into the first inner lumen 100 from the first end 101 toward the second end 102 in the direction from the third end 201 toward the fourth end 202, the direction from the second end 102 toward the third end 201 during the insertion of the guide base 2 into the first inner lumen may be defined as a positive direction. As such, for example, if the first snap 17 is closer to the second end 102 than the first snap receptacle 22, the distance between the first snap 17 and the first snap receptacle 22 will be positive. Additionally, the distance will be negative if the first snap 17 is farther away from the second end 102 than the first snap receptacle 22, or become zero when the first snap 17 and first snap receptacle 22 are equidistant from the second end 102, i.e., when the first snap 17 fits in the first snap receptacle 22. Likewise, the distance between the second snap 12 and the second snap receptacle 24 may also be directional. Since the distance between the first snap 17 and the first snap receptacle 22 is smaller than the distance between the second snap 12 and the second snap receptacle 24, as the guide base 2 moves into the first inner lumen 100, the first snap 17 will snap into the first snap receptacle 22, and then disengage from the first snap receptacle 22, concurrently with the distance between them turning negative, as a result of a further movement of the guide base 2 into the first inner lumen 100. When the guide base 2 continues to move into the first inner lumen 100, the second snap 12 will finally form a snap-in fit with the second snap receptacle 24. In this way, it is ensured that the snap-in fit between the first snap 17 and the first snap receptacle 22a precedes the snap-in fit between the second snap 12 and the second snap receptacle 24, and the latter occurs also after the disengagement of the first snap 17 from the first snap receptacle 22. The snap-in fit between the first snap 17 and the first snap receptacle22 prevents radial displacement between the guide cover 1 and the guide base 2 and limits their mutual axial displacement so that they are in a stable coaxial configuration. This facilitates the introduction of a conical tip of a delivery device and thus subsequent compression of the implant. The snap-in fit between the second snap 12 and the second snap receptacle 24 prevents radial displacement, axial displacement and circumferential rotation between the guide cover 1 and the guide base 2. Fixing the guide cover 1 with respect to the guide base 2 can prevent twisting, slanting or abrasion of an implant during compression thereof. The snap-in fits between the snaps and the respective snap receptacles allow easy engagement of the guide cover 1 with the guide base 2 and more efficient loading of the implant. It is to be noted that, the first and second snaps 17, 12 are not limited to being provided on the guide cover 1, and the first and second snap receptacles 22,24 are not limited to being provided on the guide base 2. In some other embodiments, it is also possible that the first and second snaps 17, 12 are provided on the guide base 2 and the first and second snap receptacles 22,24 on the guide cover 1. A similar effect can be obtained in accordance with such embodiments.

Preferably, the first snap 17 has a first snap surface 171 facing the second end 102 and a first engagement surface 172 facing the interior of the guide cover 1, and the first snap receptacle 22 has a second snap surface 221 facing the third end 201 and a second engagement surface 222 facing the exterior of the guide base 2. The first engagement surface 172 is configured to abut against the second engagement surface 222 so as to limit radial displacement of the guide cover 1 with respect to the guide base 2. The first snap surface 171 is configured to abut against the second snap surface 221 so as to limit axial displacement of the guide cover 1 with respect to the guide base 2 and thus snap the first snap 17 in the first snap receptacle 22. During the insertion of the guide base 2 into the first inner lumen 100, first of all, as shown in Fig. 4, the guide base 2 approaches the first inner lumen 100 in the direction from the third end 201 to the fourth end 202. Then, the fourth end 202 enters the first inner lumen 100 from the first end 101 of the guide cover 1 and continues to move toward the second end 102 of the guide cover 1 until the first snap 17 fits into the first snap receptacle 22. At this point, the loading tool for implant is in a first locked configuration, in which radial displacement of the guide cover 1 with respect to the guide base 2 is prevented, and the snap-in fit between the first snap 17 and the first snap receptacle 22 further limits axial displacement of the guide cover 1 toward the second end 102 with respect to the guide base 2. It is to be noted that, in this embodiment, axial displacement of the guide cover 1 toward the first end 101 with respect to the guide base 2 is not limited. That is, the guide cover 1 is brought into coaxial engagement with the guide base 2, which so limits the guide cover 1 that it is only unidirectionally displaceable in the axial direction. When the guide base 2 is further advanced toward the second end 102 of the guide cover 1, the first snap 17 will disengage from the first snap receptacle 22. Here, the disengagement of the first snap 17 from the first snap receptacle 22 means, in particular, that the abutment of the first snap surface 171 against the second snap surface 221 is released, with the abutment of the first engagement surface 172 against the second engagement surface 222 being retained. In other words, the disengagement of the first snap 17 from the first snap receptacle 22 means that, compared to the first locked configuration, the guide base 2 is moveable in the first inner lumen 100 toward the second end 102, while radial displacement of the guide cover 1 with respect to the guide base 2 remains limited by the abutment of the first engagement surface 172 against the second engagement surface 222. With this arrangement, the guide cover 1 and guide base 2 can be constantly maintained coaxial with each other, thus facilitating the introduction of a conical tip of a delivery device and thus subsequent compression of the implant. It is to be noted that while limiting axial replacement of the guide cover 1 with respect to the guide base 2 has been described above as being accomplished by the abutment of the first snap surface 171 of the first snap 17 against the second snap surface 221 of the first snap receptacle 22 in this embodiment. In other embodiments, the snap-in fit between the first snap 17 and the first snap receptacle 22 may also be accomplished by other means such as a friction-based snap-in fit or a snap-in fit between a groove and a protraction so as to achieve a limited axial replacement of the guide cover 1 with respect to the guide base 2.

More preferably, the first snap 17 is provided on one of the inter-slot portions 181 in the first section 13, and as shown in Fig. 4, and the first snap receptacle 22 is provided, along the direction facing the fourth end 202, a sloped surface circumferentially around the guide base 2. The sloped surface gradually expands in the direction from the fourth end 202 toward the third end 201, thus forming a flare having a maximum outer diameter that is greater than an outer diameter of the second engagement surface 222. At the same time, an inner diameter of the first engagement surface 172 is preferably equal to or slightly smaller than the outer diameter of the second engagement surface 222. As such, a complementary or interference fit can be formed. As the first section 13 of the guide cover 1 is circumferentially divided into multiple sub-sections by the plurality of slots 180, during the insertion of the guide base 2 into the first inner lumen 100 of the guide cover 1, the sub-sections all tend to elastically deform under the guidance of the sloped surface and hence extend radially outward from the guide cover 1 until the first engagement surface 172 abuts against the second engagement surface 222 and thus limits radial displacement of the guide cover 1 with respect to the guide base 2. As will be appreciated, the number of the slots 180 may vary as required, for example, from 2 to 12. Preferably, the slots 180 are uniformly distributed circumferentially around the guide cover 1 so that the inter-slot portions 181 have a symmetrical distribution circumferentially around the guide cover 1, such as an axisymmetric distribution, a centrosymmetric distribution or a rotationally symmetric distribution. Alternatively, all, or preferably, some of the inter-slot portions 181 may be each provided with such a first snap 17. This can not only ensure coaxial engagement but can facilitate detachment. More preferably, the first snaps 17 have a centrosymmetric distribution circumferentially around the guide cover 1, which ensures the guide cover 1 and the guide base 2 engage with each other with uniform stressing and no slanting.

Additionally, the second snap 12 includes a plurality of second snap fasteners, and the second snap receptacle 24 includes a plurality of second snap recesses for engaging the respective second snap fasteners. The second snap recesses are configured to limit: radial displacement and circumferential rotation of the guide cover 1 with respect to the guide base 2 during the insertion of the guide base 2 into the first inner lumen 100; and radial and axial displacement and circumferential rotation of the guide cover 1 with respect to the guide base 2 after the guide base 2 has been inserted in the first inner lumen 100. The engagement between the second snap fasteners and the second snap recesses may be active, preferably elastic, engagement therebetween. In particular, the second snap fasteners may be also provided on respective ones of the inter-slot portions 181, like the case shown in Figs. 1 and 2, in which two second snap fasteners are provided on two respective ones of the inter-slot portions 181, which are arranged in symmetry with each other with respect to the axis of the guide cover 1.

Preferably, each of the second snap fasteners has a first bevel 121, and each of the second snap recesses has a second bevel 241 for engaging the first bevel 121 of a respective one of the second snap fasteners. The first bevel 121 is inclined at an angle with respect to the axial direction of the guide cover 1, and the first bevel 121 is preferably tapered in the direction from the second end 102 to the first end 101. In order to easily guide the second snap fasteners into the respective second snap recesses, the angle may be selected to be, for example, greater than 0° and smaller than 60°, more preferably, greater than 0° and smaller than 30°. During the insertion of the guide base 2 into the first inner lumen 100, the second bevels 241 abut against the respective first bevel 121, thus limiting radial displacement of the guide cover 1 with respect to the guide base 2. Each of the second snap fasteners has third stop surfaces 123 arranged along the circumference of the guide cover 1, and each of the second snap recesses has fourth stop surfaces 243 for engaging the respective third stop surfaces 123 of a respective one of the second snap fasteners. The third stop surfaces 123 abut against the respective fourth stop surfaces 243, so that circumferential rotation of the guide cover 1 with respect to the guide base 2 is limited. Each of the second snap fasteners has a third snap surface 122 facing the second end 102, and each of the second snap recesses has a fourth snap surface 242 for engaging the third snap surface 122. When the guide base 2 has been inserted in the first inner lumen 100, the third snap surfaces 122 abut against the respective fourth snap surfaces 242, thus limiting axial displacement of the guide cover 1 with respect to the guide base 2. As shown in Figs. 4 and 5, each of the second snap recesses has a pocket 244 that is complementary in shape with, and configured for insertion therein of, a respective one of the second snap fasteners. As shown in Fig. 5, the second bevel 241 and the fourth stop surfaces 243 form internal surfaces of the pocket 244, and the two fourth stop surfaces 243 oppose each other. Correspondingly, the two third stop surfaces 123 form side surfaces of the second snap fastener along the circumference of the guide cover 1. When the second snap fasteners are inserted in the respective pockets 244 of the respective second snap recesses, the side surfaces of each second snap fastener will abut against the respective fourth stop surfaces 243 of a respective one of the pockets 244, thus limiting circumferential rotation of the guide cover 1 with respect to the guide base 2. As shown in Fig. 7, the second bevels 241 are configured to guide the respective first bevels 121. Specifically, during the insertion of the guide base 2 into the first inner lumen 100, the second bevels 241 will come into abutment against the respective first bevels 121. Since each of the first bevels 121 and the respective complementary second bevels 241 is inclined at an angle with respect to the axial direction of the guide cover 1, and because the inner diameter at each first bevel 121 gradually increases in the direction from the first end 101 to the second end 102, i.e., each of the first and second bevels 121, 241 is inclined inward along the direction in which the second snap fasteners are inserted into the respective second snap recesses, a portion of each second snap fastener proximal to the first end 101 forms a tapered wedge-like shape. With this arrangement, on the one hand, since the second bevels 241 are inclined inward in the direction toward the third end 201, they will exert, on the respective first bevels 121, force components radially toward the inside of the guide base 2, as the second snap fasteners are being inserted into the respective second snap recesses, causing some deformation of the second snap fasteners. At the same time, the second snap fasteners will produce reaction forces, which enable reliable abutment of the first bevels 121 against the respective second bevels 241, thus ensuring prevention of radial displacement of the guide cover 1 with respect to the guide base 2. On the other hand, as a result of elastic deformation of the second snap fasteners, the second bevels 241 also exert, on the respective first bevels 121, axial force components toward the fourth end 202, which contribute to prevention of axial movement of the second snap fasteners toward the third end 201 of the guide base 2 and, coupled with the aforementioned snap-in fit between the first snap 17 and the first snap receptacle 22, allow good abutment of the first snap surface 171 against the second snap surface 221 even when under the action of an external force smaller than the axial direction force components toward the fourth end 202. Thus, better prevention of axial displacement of the guide cover 1 with respect to the guide base 2 can be achieved in the first locked configuration of the guide cover 1 and the guide base 2.

Further, each of the second snap fasteners has a third snap surface 122 facing the second end 102, and each of the second snap recesses has a fourth snap surface 242 for engaging the third snap surface 122. When the guide base 2 has been inserted in the first inner lumen 100, the third snap surfaces 122 abut against the respective fourth snap surfaces 242, thus limiting axial displacement of the guide cover 1 with respect to the guide base 2. With the guide base 2 having been inserted in the first inner lumen 100, the guide cover 1 is securely assembled with the guide base 2. At this point, they are in a second locked configuration, in which the fourth end 202 protrudes beyond the second end 102 along the direction from the first end 101 toward the second end 102 (to this end, the guide base 2 may have an outer diameter at the fourth end 202, which is smaller than an inner diameter of the guide cover 1 at the second end 102). As shown in Figs. 6 and 7, the first bevels 121 have disengaged from the respective second bevels 241 and are positioned closer to the third end 201 compared with the respective second bevels 241, and the third snap surfaces 122 abut against the respective fourth snap surfaces 242, thereby limiting axial displacement of the guide cover 1 with respect to the guide base 2. Here, it is to be noted that the prevention of axial displacement of the guide cover 1 with respect to the guide base 2 accomplished by the abutment of the third snap surfaces 122 against the respective fourth snap surfaces 242 refers to prevention of axial displacement of the guide cover 1 with respect to the guide base 2 toward the second end 102. Prevention of axial displacement of the guide cover 1 with respect to the guide base 2 toward the first end 101 may be accomplished by other components of the guide cover 1 and the guide base 2, for example, limiting shoulders provided respectively on the guide cover 1 and the guide base 2, which may act in a direction opposite to that of the third and fourth snap surfaces 122, 242 and come into abutment against each other at the same time as the abutment of the third and fourth snap surfaces 122, 242. In this way, axial displacement of the guide cover 1 with respect to the guide base 2 can be prevented in both directions. Of course, the prevention of axial displacement between the guide cover 1 and the guide base 2 is not limited to being accomplished in the manner as described above, as it may be accomplished otherwise, for example, without limitation, by friction.

Furthermore, each of the second snap fasteners has a fifth stop surface 124 facing the outside of the guide cover 1. Upon the third snap surfaces 122 coming into abutment against the respective fourth snap surfaces 242, the fifth stop surfaces 124 will come into abutment against the respective second bevels 241 (especially portions thereof proximal to the third end 201), thus additionally contributing to the prevention of radial displacement of the guide cover 1 with respect to the guide base 2. Preferably, an outer diameter at the fifth stop surfaces 124 is not smaller than a minimum inner diameter at the second bevels 241 (i.e., at the portions thereof proximal to the third end 201). In particular, when the outer diameter at the fifth stop surfaces 124 is slightly greater than the minimum inner diameter at the second bevels 241, better prevention of radial displacement of the guide cover 1 with respect to the guide base 2 can be achieved by virtue of reaction forces produced by the second snap fasteners from their deformation.

Furthermore, the third snap surfaces 122 of the guide cover 1 are oriented the same as the first snap surface 171 and the fourth snap surfaces 242 of the guide base 2 as the second snap surface 221. During the insertion of the guide base 2 into the first inner lumen 100, the distance between the second snap surface 221 and the first snap surface 171 is always smaller than the distance between each of the fourth snap surfaces 242 and a respective one of the third snap surfaces 122. This can ensure that the snap-in fit between the first snap 17 and the first snap receptacle 22a precedes the snap-in fit between the second snap 12 and the second snap receptacle 24, and the latter occurs also after the first snap 17 has disengaged from the first snap receptacle 22.

Preferably, the distance between a bottom of the second snap receptacle that faces the fourth end 202 and a top of the second snap 12 that faces the first end 101 is smaller than the distance between the first snap 17 and the first snap receptacle 22 (here, the distances are measured along the axial directions of the guide cover 1 and the guide base 2 and are both directional, and since the guide base 2 is inserted into the first inner lumen 100 from the first end 101 toward the second end 102 in the direction from the third end 201 to the fourth end 202, the direction from the second end 102 to the third end 201 can be defined as a positive direction for the insertion of the guide base 2 into the first inner lumen 100). With this arrangement, radial and axial displacement and circumferential rotation between the guide cover 1 and the guide base 2 are prevented when the first snap 17 is snapped in the first snap receptacle 22, with the second snap 12 being partially received in the second snap receptacle and a first snap-in fit achieved.

In the second locked configuration of the guide cover 1 and the guide base 2, the abutment of the third snap surfaces 122 against the respective fourth snap surfaces 242 can be released by pressing the second snap 12 toward the inside of the guide cover 1. The second locked configuration of the guide cover 1 and the guide base 2 can be then released by pushing the second snap 12 toward the second end 102 of the guide cover 1. Thereafter, the first locked configuration of the guide cover 1 and the guide base 2 can be further released, leading to disengagement of them, by pushing the guide cover 1 toward the second end 102, or by pulling the guide base 2 toward the third end 201 (or by both).

In some other embodiments, the number of second snap fasteners and that of second snap recesses are not limited to two, and each of the second snap fasteners is adjacent to, and spaced by slots 180 from, on both sides to different inter-slot portions181. In this case, opposing side faces of each second snap fastener that interface with the respective slots 180 may provide the aforementioned third stop surfaces 123 thereof. The plurality of second snap fasteners may be evenly distributed circumferentially around the guide cover 1, and the plurality of second snap recesses may be evenly distributed circumferentially around the guide base 2. Such even distributions of the second snap fasteners and recesses allow the guide cover 1 and the guide base 2 to engage with each other with more uniform stressing and reduced slanting. The number of second snap fasteners and that of second snap recesses may be determined as required, and the present invention is not limited in this regard.

As shown in Figs. 2 and3, the first inner lumen 100 of the guide cover 1 is divided into a first section 13, a second section 16, a third section 15 and a fourth section 14, which are sequentially joined together from the first end 101 toward the second end 102. An inner diameter of the second section 16 at an end thereof closer to the first end 101 (i.e., at the cross-section *d*) that is greater than an inner diameter of the fourth section 14 at an end thereof closer to the second end 102 (i.e., at the cross-section *a*). An inner diameter of the third section 15 at an end thereof closer to the first end 101 (i.e., at the cross-section *c*) and an inner diameter of the third section 15 at an end thereof closer to the second end 102 (i.e., at the cross-section *b*) may be equal to each other or not, and both of them are smaller than the inner diameter of the second section 16 at the end thereof closer to the first end 101 (i.e., at the cross-section *d*) and greater than the inner diameter of the fourth section 14 at the end thereof closer to the second end 102 (i.e., at the cross-section *a*). In practical use, the second, third and fourth sections 16, 15, 14 are configured to compress an implant.

Preferably, the first, second, third and fourth sections 13, 16, 15, 14 all have circular cross-sections and straight generatrices (in geometry, a generatrix is a curve that, when moved along a given path, generates a curved surface), as shown in Fig. 2. Therefore, the first section 13 is a cylinder, and the second section 16 is a conical frustum with its larger base facing the first end 101. The third section 15 is a cylinder or conical frustum, and the fourth section 14 is a conical frustum with its larger base facing the first end 101. More preferably, the generatrices of the first, second, third and fourth sections 13, 16, 15, 14 continue smoothly from one to another, imparting good ability of the first inner lumen 100 to compress an implant. Of course, without limitation, instead of continuing smoothly from one to another, it is also possible that the generatrices of the first, second, third and fourth sections 13, 16, 15, 14 are directly joined together in a non-smooth way with angles formed at the joints, or that any two of them continue smoothly from one to the other.

In some other embodiments, the first, second, third and fourth sections 13, 16, 15, 14 may all have circular cross-sections, with the generatrices of the third and fourth sections 15,14 being curved and the generatrix of the third section 15 smoothly continuing with both the generatrices of the fourth and second sections 14,16, as shown in Fig. 3. The generatrix of the third section 15 may bulge toward the interior of the guide cover 1, which is helpful in securing of an implant under a condition with no external force and thus the operator's correction of positional offsets of the implant during its compression. Correspondingly, the generatrix of the fourth section 14 may also be curved, for example, bulging toward the exterior of the guide cover 1. Of course, without limitation, the generatrix of the fourth section 14 may be alternatively a straight line.

It is to be noted that, as used herein, the term "gradual decreasing" of a cross-sectional area of the first inner lumen 100 from the first end 101 to the second end 102 means an overall cross-sectional tapering of the first inner lumen 100 from the first end 101 to the second end 102, but not that a cross-sectional area of the first inner lumen 100 at any point along its length must be greater than a cross-sectional area of the first inner lumen 100 at a point that is closer to the second end 102. For example, in case of the generatrix of the third section 15 bulging toward the inside of the guide cover 1, it is possible for the third section 15 to have a cross-sectional area at a corresponding inward bulging portion that is greater than a cross-sectional area thereof at a point closer to the first end 101. In this case, the third section 15 is still considered as overall tapering from the first end 101 to the second end 102. Accordingly, the first inner lumen 100 is considered as gradually decreasing in cross-section from the first end 101 to the second end 102.

Referring to Fig. 5, the guide base 2 has a second inner lumen 200 axially extending therethrough, which gradually decreases in cross-sectional area from the third end 201 to the fourth end 202. Similarly, the "gradual decreasing" of the second inner lumen 200 in cross-sectional area from the third end 201 to the fourth end 202 here also refers to an overall tapering without limitation. Preferably, the second inner lumen 200 sequentially includes a fifth section 26 and a sixth section 25, which are joined from the third end 201 to the fourth end 202. An inner diameter of the fifth section 26 at an end thereof closer to the third end 201 (i.e., at the cross-section h) is greater than an inner diameter of the fifth section 26 at an end thereof closer to the fourth end 202 (i.e., at the cross-section *g*). An inner diameter of the sixth section 25 at an end thereof closer to the third end 201 (i.e., at the cross-section g) is equal to the inner diameter of the fifth section 26 at the end closer to the fourth end 202 and is not smaller than an inner diameter of the sixth section 25 at an end thereof closer to the fourth end 202 (i.e., at the cross-section *f*).In practice, the fifth section 26 is configured to compress an implant.

Preferably, both the fifth and sixth sections 26,25 have circular cross-sections and straight generatrices. Therefore, the fifth section 26 is a conical frustum with its larger base facing the third end 201, and the fifth section 26 is a cylinder or conical frustum. More preferably, the generatrices of the fifth and sixth sections 26, 25 smoothly continue from one to the other, thus similarly imparting good ability of the second inner lumen 200 to compress an implant. Of course, instead of continuing smoothly from one to the other, it is also possible that the generatrices of the fifth and sixth sections 26,25 are directly joined together in a non-smooth way with angles formed at the joints, or that either or both of these generatrices is/are curved. For example, the generatrix of the sixth section 25 may be a smoothly transitioning curve, or a combination of straight and curved lines. This can also achieve a good effect, and the present invention is not limited in this regard.

In addition, as shown in Fig. 5, an outer circumference of the guide base 2 is provided with anchoring trenches 23 with notches, and the notches of the anchoring trenches 23 are oriented toward the fourth end 202. The anchoring trench 23 is configured to limit axial displacement of an implant toward the third end 201 by receiving an end portion of the implant so that the implant abuts against an internal bottom surface of the trench.

Preferably, anti-slip features 19 for facilitating gripping are provided on or in outer circumferential walls of both the guide cover 1 and the guide base 2. As shown in Fig. 1, anti-slip features 19 may be provided on or in an outer surface of the guide cover 1, which may be, for example, annular anti-slip ridges extending circumferentially around the guide cover 1. As shown in Fig. 4, anti-slip features 19 may be provided on or in an outer surface of the guide base 2, which may be, for example, anti-slip grooves or the like. Additionally, anti-slip features may also be provided on or in the first bevels121, such as concave anti-slip grooves, convex anti-slip teeth, etc. The present invention is not limited to any particular type of the anti-slip features, and anti-slip features of any type are possible as long as they can facilitate gripping of, or increase friction with, the operator.

More preferably, the guide cover 1 is formed of a transparent material, such as polycarbonate (PC), and provided with an indicator ring 11, which is arranged circumferentially around the guide cover 1 and is configured to indicate the progress in loading an implant. The indicator ring 11 is capable of indicating that a predetermined degree of compression of the implant in the loading tool has been reached and it is ready to perform the subsequent operations, and may be in the form of a paint mark, a groove, a ridge or any other readily noticeable mark. Moreover, it may be used in combination with a sound or light from an external handle to provide the operator with an indication. The indicator ring 11 may be positioned depending on an initial degree of compression of the implant, and is not limited to having an annular shape as it may alternatively have for example, a dot- or block-based shape. During the compression and loading of the implant, when it reaches the position of the indicator ring 11, the guide cover 1 and guide base 2 may be disengaged to allow the subsequent operations. It is to be noted that the guide cover 1 is not limited to being entirely made of the transparent material, as it is also possible that only part of the guide cover 1, for example, the second through fourth sections 16-14, is made of the transparent material. The guide cover 1 may be either an integral part or a welded, fused or bonded assembly of multiple components. For example, without limitation, the first section 13 and the second snap 12 may be fabricated from a material with good elasticity and then securely assembled with the second through fourth sections 16-14 made of the transparent material.

Referring to Figs. 8 to 13, in the present embodiments, there is also provided a medical device comprised of the above loading tool for implant and a delivery device 5. The loading tool for implant is configured to work with the delivery device 5 to load an implant into the delivery device 5. The implant is a compressible implant, such as a heart valve stent. The present invention is not limited to any particular shape or material of the implant, and any suitable existing implant is possible.

With the implant being a valve stent 9 as an example, how the loading tool for implant is used and how the medical device is structured and operates will be explained below with reference to the accompanying drawings.

Referring to Fig. 13, the valve stent 9 includes an outflow section92, an inflow section93 and attachment projections 91, and the loading tool discussed above is used to compress and crimp the valve stent 9 with the aid of the delivery device 5 and load it into the delivery device 5. Subsequently, the valve stent 9 is delivered in the crimped state through a catheter of the delivery device 5 to a target site in a patient's body and released there. After released, the valve stent 9 expands to take on a configuration as shown in Fig. 13.

Referring to Fig. 8, the delivery device 5 includes a conical tip 54, an anchor 55, a sheath 56, a catheter and a handle (not shown). The conical tip 54 is fixed to the anchor 55 which is received within the sheath 56. The anchor 55 is secured to the handle by a connecting member, and the handle can be manipulated to displace the sheath 56so that the anchor 55 is exposed to allow the attachment projections 91 of the valve stent 9 to be attached thereto and thus serve as points of force application to the valve stent 9.

During use of the loading tool for implant, the valve stent 9 is placed with its inflow section93 received within the anchoring trench 23 in the guide base 2, and the guide cover 1 is then pressed from the outflow section92 of the valve stent 9 toward the guide base 2 (i.e., in the direction of insertion of the guide base 2 into the first inner lumen 100) so that a snap-in fit is formed between the first snap 17 of the guide cover 1 and the first snap receptacle 22 of the guide base 2,which disallows further relative axial movement between the guide cover 1 and the guide base 2. In this process, limited by the anchoring trench 23 at one end, the valve stent 9 is preliminarily compressed by the second section 16 of the guide cover 1, concurrently with its outflow section 92 thereof sliding into the third section 15. As a result of the snap-in fit being formed between the first snap 17 and the first snap receptacle 22, the guide cover 1 and guide base 2 form the aforementioned first locked configuration, where the guide cover 1, the guide base 2 and the valve stent 9 are stably maintained coaxial with one another, making it easier to introduce the conical tip 54 to allow further compression.

As shown in Fig. 4, the conical tip 54 is advanced into the second inner lumen 200 from the fourth end 202 of the guide base 2 (in this process, the guide cover 1 and the guide base 2 are in the first locked configuration, and the valve stent 9 is disposed between the guide cover 1 and the guide base 2), and the attachment projections 91 are roughly aligned with the anchor 55. Subsequently, the guide cover 1 is further pushed toward the guide base 2 until the second snap 12 engages the second snap receptacle24. As a result, the valve stent 9 is advanced from the third section 15 into the fourth section 14 and then partially out of the fourth section 14, and the guide cover 1, the guide base 2 and the valve stent 9 are secured against one another, allowing the attachment projections 91 to be secured in respective recesses in the anchor 55 as shown in Figs. 9 and 10. After confirming that the attachment projections 91 have been secured in the respective recesses, the handle is manipulated to advance the sheath 56 toward the inflow section 93 (namely, toward the third end 201 of the guide base 2, i.e., to the right as viewed in the orientation of the figures) so that the valve stent 9 enters the sheath 56 and starts to move forward therein, thus beginning the process of loading the valve stent 9 into the sheath 56. This process can be monitored through the transparent guide cover 1, and the end of a first loading phase can be indicated by the arrival or approach of a bottom of the inflow section 93 (namely, the lower end thereof as viewed in the orientation of Fig. 13) at or to the indicator ring 11 of the guide cover 1. In the first loading phase, the guide cover 1 can be radially, circumferentially and axially immobilized with respect to the guide base 2 to effectively prevent twisting, slanting or abrasion of the valve stent 9 that is being compressed, enabling the compression to proceed in a smooth way.

Referring to Figs. 11 and 12, at the end of the first loading phase, the second snap 12 is pressed to disengage the guide cover 1 from the guide base 2, and the guide cover 1 is moved to the left as viewed in the orientation of the figures so that the valve stent 9 is exposed (not shown). The guide base 2 is then flipped over (i.e., the third and fourth ends 201, 202 are turned to face the respective opposite directions) and is moved toward the sheath 56 in the direction from the fourth end 202 to the third end 201 (i.e., to the left as viewed in the orientation of the figures). As a result, pushed by the fifth section 26 of the guide base 2, the inflow section 93 of the valve stent 9 is advanced into the sixth section 25, so that the inflow section 93 is reduced to an outer diameter that is close to an inner diameter of the sheath 56. Afterward, the handle is again manipulated to push the sheath 56 forward, thus advancing the valve stent 9 to the left as viewed in the orientation of the figures. In this way, the valve stent 9 is completely loaded into the sheath 56, marking the end of the loading process.

In summary, during loading of the valve stent 9 into the loading tool for implant of the present invention, the guide cover 1 and the guide base 2 undergo two interlocking processes (corresponding to the first and second locked configurations). The first interlocking process involves engagement of the first snap 17 with the first snap receptacle 22, which brings the guide cover 1, the guide base 2 and valve stent 9 into a stable coaxial configuration facilitating the introduction of the conical tip 54 and hence subsequent operations. The second interlocking process involves engagement of the second snap 12 with the second snap receptacle 24, which immobilizes the guide cover 1 with respect to the guide base 2, radially, circumferentially and axially, helping in preventing twisting, slanting or abrasion during compression of the valve stent 9. This design allows firmer securing, can better ensure that the valve stent 9, the first inner lumen 100 of the guide cover 1, the second inner lumen 200 of the guide base 2 and sheath 56 are maintained coaxial with respect to each other throughout the compression process, and prevents twisting or slanting of the valve stent 9 during the process. Further, securing the guide cover 1 and the guide base 2 against each other by snap-in fits can not only ensure the immobilization of the guide cover 1 with respect to the guide base 2 throughout the first phase of loading the valve stent 9, but also allows easy unlocking for the subsequent operations. Thus, the whole process is simple and controllable and provides improved loading efficiency.

## Claims

1. A loading tool for an implant, comprising a guide cover (1) and a guide base (2) detachably coupled to the guide cover (1), wherein: the guide cover (1) has a first end (101) and a second end (102) opposing the first end(101) along an axial direction thereof; the guide base (2) has a third end (201) and a fourth end (202) opposing the third end (201) along an axial direction thereof; the guide cover (1) has a first inner lumen (100) extending therethrough; and the guide base (2) is configured for inserting into the first inner lumen (100) from the first end (101) toward the second end (102) in a direction from the third end (201) toward the fourth end (202),
wherein one of the guide cover (1) and the guide base (2) is provided with a first snap (17) and a second snap (12), and the other one of the guide cover (1) and the guide base (2) is provided with a first snap receptacle (22) and a second snap receptacle (24), the first snap (17) configured to form a snap-in fit with the first snap receptacle (22) to limit a radial and axial displacement of the guide cover (1) with respect to the guide base (2), the second snap (12) configured to form a snap-in fit with the second snap receptacle (24) to limit a radial and axial displacement and a circumferential rotation of the guide cover (1) with respect to the guide base (2), and
wherein during the insertion of the guide base (2) into the first inner lumen (100), a distance between the first snap (17) and the first snap receptacle (22) is smaller than a distance between the second snap (12) and the second snap receptacle (24).

2. The loading tool for an implant of claim 1, wherein the first and second snaps (17,12) are provided on the guide cover (1), and the first and second snap receptacles (22,24) are provided on the guide base (2), wherein the first snap (17) has a first snap surface (171) facing the second end (102) and a first engagement surface (172) facing an interior of the guide cover (1), wherein the first snap receptacle (22) has a second snap surface (221) facing the third end (201) and a second engagement surface (222) facing an exterior of the guide base (2), and wherein the first engagement surface (172) is configured to abut against the second engagement surface (222) so as to limit the radial displacement of the guide cover (1) with respect to the guide base (2), and the first snap surface (171) is configured to abut against the second snap surface (221) so as to limit the axial displacement of the guide cover (1) with respect to the guide base (2), and thus to form the snap-in fit between the first snap (17) and the first snap receptacle (22).

3. The loading tool for an implant of claim 2, wherein the second snap (12) comprises a plurality of second snap fasteners and the second snap receptacle (24) comprises a plurality of second snap recesses configured for engagement with respective second snap fasteners, and wherein the second snap recesses are configured to limit:
the radial displacement and the circumferential rotation of the guide cover (1) with respect to the guide base (2) during the insertion of the guide base (2) into the first inner lumen (100); and
the radial and axial displacement and the circumferential rotation of the guide cover (1) with respect to the guide base (2) after the guide base (2) has been inserted in the first inner lumen (100).

4. The loading tool for an implant of claim 3, wherein each of the plurality of second snap fasteners has a first bevel (121) preferably provided with an anti-slip groove, and each of the plurality of second snap recesses has a second bevel (241) for engaging a corresponding first bevel (121), wherein the first bevel (121) is inclined at an angle with respect to the axial direction of the guide cover (1), wherein the second bevel (241) is configured to abut against the first bevel (121) during the insertion of the guide base (2) into the first inner lumen (100).

5. The loading tool for an implant of claim 3, wherein each of the plurality of second snap fasteners has a third stop surface (123) arranged along a circumference of the guide cover (1) and each of the plurality of second snap recesses has a fourth stop surface (243) for engaging a corresponding third stop surface (123), and wherein the third stop surfaces (123) are configured to abut against the corresponding fourth stop surfaces (243) so as to limit the circumferential rotation of the guide cover (1) with respect to the guide base (2); and/or
each of the plurality of second snap fasteners has a third snap surface (122) facing the second end (102) and each of the plurality of second snap recesses has a fourth snap surface (242) for engaging a corresponding third snap surface (122), and wherein the third snap surface (122) is configured to abut against the fourth snap surface (242) after the guide base (2) has been inserted in the first inner lumen (100) so as to limit the axial displacement of the guide cover (1) with respect to the guide base (2).

6. The loading tool for an implant of claim 3, wherein the plurality of second snap fasteners are evenly distributed around a circumference of the guide cover (1), and wherein the plurality of second snap recesses are evenly distributed around a circumference of the guide base (2).

7. The loading tool for an implant of claim 3, wherein with the snap-in fit between the first snap (17) and the first snap receptacle (22) being formed, each of the second snap fasteners is partially received in a corresponding second snap recess, thereby limiting the axial and radial displacement and the circumferential rotation of the guide cover (1) with respect to the guide base (2).

8. The loading tool for an implant of claim 1, wherein the first inner lumen (100) has a cross-sectional area gradually decreased from the first end (101) to the second end (102), and preferably
the first inner lumen (100) comprises a first section (13), a second section (16), a third section (15) and a fourth section (14), which are sequentially joined from the first end (101) to the second end (102),
wherein the first section (13) has a constant inner diameter along the axial direction of the guide cover (1), and wherein an end portion of the first section (13) close to the first end (101) has a plurality of slots (180) formed along a circumferential direction,
wherein the second section (16) has an inner diameter at an end thereof closer to the first end (101) being greater than an inner diameter of the fourth section (14) at an end thereof closer to the second end (102),
wherein each of an inner diameter of the third section (15) at an end thereof closer to the first end (101) and an inner diameter of the third section (15) at an end thereof closer to the second end (102) is smaller than the inner diameter of the second section (16) at the end thereof closer to the first end (101) and is greater than the inner diameter of the fourth section (14) at the end thereof closer to the second end (102).

9. The loading tool for an implant of claim 1, wherein the guide base (2) has a second inner lumen (200) extending therethrough along the axial direction of the guide base (2), and wherein the second inner lumen (200) has a cross-sectional area gradually decreased from the third end (201) to the fourth end (202), and preferably
the second inner lumen (200) sequentially comprises a fifth section (26) and a sixth section (25), which are joined from the third end to the fourth end,
wherein the fifth section (26) has an inner diameter at an end thereof closer to the third end (201) being greater than an inner diameter of the fifth section (26) at an end thereof closer to the fourth end (202), and
wherein the sixth section (25) has an inner diameter at an end thereof closer to the third end (201) being equal to the inner diameter of the fifth section (26) at the end thereof closer to the fourth end (202), and being not smaller than an inner diameter of the sixth section (25) at an end thereof closer to the fourth end (202).

10. The loading tool for an implant of claim 1, wherein an outer circumference of the guide base (2) is provided with anchoring trenches (23) with notches, and wherein the notches of the anchoring trenches (23) are oriented toward the fourth end (202).

11. The loading tool for an implant of claim 1, wherein with the snap-in fit between the second snap (12) and the second snap receptacle (24) being formed, the fourth end (202) protrudes beyond the second end (102) along a direction from the first end (101) toward the second end (102).

12. The loading tool for an implant of claim 1, wherein each of the guide cover (1) and the guide base (2) is provided with anti-slip features (19) on or in its outer circumferential wall for facilitating gripping.

13. The loading tool for an implant of any one of claims 1 to 12, wherein the guide cover (1) is formed of a transparent material, and wherein the guide cover (1) is provided with an indicator ring (11) arranged circumferentially around the guide cover (1) to indicate a progress in loading an implant.

14. A medical device comprising the loading tool for an implant as defined in any one of claims 1 to 13 and a delivery device (5), wherein the loading tool is configured to cooperate with the delivery device (5) to load an implant into the delivery device.

15. The medical device of claim 14, wherein the implant is a valve stent (9).

## Patentansprüche

1. Ladewerkzeug für ein Implantat, umfassend eine Führungsabdeckung (1) und eine Führungsbasis (2), die abnehmbar an die Führungsabdeckung (1) gekoppelt ist, wobei: die Führungsabdeckung (1) ein erstes Ende (101) und ein zweites Ende (102) gegenüber dem ersten Ende (101) entlang einer axialen Richtung davon aufweist; die Führungsbasis (2) ein drittes Ende (201) und ein viertes Ende (202) gegenüber dem dritten Ende (201) entlang einer axialen Richtung davon aufweist; die Führungsabdeckung (1) ein erstes inneres Lumen (100) aufweist, das sich durch sie hindurch erstreckt; und die Führungsbasis (2) konfiguriert ist, um von dem ersten Ende (101) zu dem zweiten Ende (102) in einer Richtung von dem dritten Ende (201) zu dem vierten Ende (202) in das erste innere Lumen (100) eingeführt zu werden,
wobei eine von der Führungsabdeckung (1) und der Führungsbasis (2) mit einer ersten Raste (17) und einer zweiten Raste (12) versehen ist und die andere von der Führungsabdeckung (1) und der Führungsbasis (2) mit einer ersten Rastaufnahme (22) und einer zweiten Rastaufnahme (24) versehen ist, wobei die erste Raste (17) konfiguriert ist, um eine Einrastpassung mit der ersten Rastaufnahme (22) zu bilden, um eine radiale und axiale Verschiebung der Führungsabdeckung (1) in Bezug auf die Führungsbasis (2) zu begrenzen, die zweite Raste (12) konfiguriert ist, um eine Einrastpassung mit der zweiten Rastaufnahme (24) zu bilden, um eine radiale und axiale Verschiebung und eine Umfangsdrehung der Führungsabdeckung (1) in Bezug auf die Führungsbasis (2) zu begrenzen, und
wobei während des Einführens der Führungsbasis (2) in das erste innere Lumen (100) ein Abstand zwischen der ersten Raste (17) und der ersten Rastaufnahme (22) kleiner ist als ein Abstand zwischen der zweiten Raste (12) und der zweiten Rastaufnahme (24).

2. Ladewerkzeug für ein Implantat nach Anspruch 1, wobei die erste und die zweite Raste (17, 12) an der Führungsabdeckung (1) bereitgestellt sind und die erste und die zweite Rastaufnahme (22, 24) an der Führungsbasis (2) bereitgestellt sind, wobei die erste Raste (17) eine erste Rastfläche (171), die dem zweiten Ende (102) zugewandt ist, und eine erste Eingriffsfläche (172), die der Innenseite der Führungsabdeckung (1) zugewandt ist, aufweist, wobei die erste Rastaufnahme (22) eine zweite Rastfläche (221), die dem dritten Ende (201) zugewandt ist, und eine zweite Eingriffsfläche (222), die einer Außenseite der Führungsbasis (2) zugewandt ist, aufweist, und wobei die erste Eingriffsfläche (172) konfiguriert ist, um an die zweite Eingriffsfläche (222) anzustoßen, um die radiale Verschiebung der Führungsabdeckung (1) in Bezug auf die Führungsbasis (2) zu begrenzen, und die erste Rastfläche (171) konfiguriert ist, um an die zweite Rastfläche (221) anzustoßen, um die axiale Verschiebung der Führungsabdeckung (1) in Bezug auf die Führungsbasis (2) zu begrenzen und somit die Einrastpassung zwischen der ersten Raste (17) und der ersten Rastaufnahme (22) zu bilden.

3. Ladewerkzeug für ein Implantat nach Anspruch 2, wobei die zweite Raste (12) eine Vielzahl von zweiten Rastbefestigungsmitteln umfasst und die zweite Rastaufnahme (24) eine Vielzahl von zweiten Rastaussparungen umfasst, die für einen Eingriff mit jeweiligen zweiten Rastbefestigungsmittel konfiguriert sind, und wobei die zweiten Rastaussparungen konfiguriert sind, um Folgendes zu begrenzen:
die radiale Verschiebung und die Umfangsdrehung der Führungsabdeckung (1) in Bezug auf die Führungsbasis (2) während des Einführens der Führungsbasis (2) in das erste innere Lumen (100); und
die radiale und axiale Verschiebung und die Umfangsdrehung der Führungsabdeckung (1) in Bezug auf die Führungsbasis (2), nachdem die Führungsbasis (2) in das erste innere Lumen (100) eingeführt worden ist.

4. Ladewerkzeug für ein Implantat nach Anspruch 3, wobei jedes der Vielzahl von zweiten Rastbefestigungsmitteln eine erste Abschrägung (121) aufweist, die vorzugsweise mit einer Abrutschsicherungsnut versehen ist, und jede der Vielzahl von zweiten Rastaussparungen eine zweite Abschrägung (241) zum Eingriff mit einer entsprechenden ersten Abschrägung (121) aufweist, wobei die erste Abschrägung (121) in einem Winkel in Bezug auf die axiale Richtung der Führungsabdeckung (1) geneigt ist, wobei die zweite Abschrägung (241) konfiguriert ist, um während des Einführens der Führungsbasis (2) in das erste innere Lumen (100) an der ersten Abschrägung (121) anzuliegen.

5. Ladewerkzeug für ein Implantat nach Anspruch 3, wobei jedes der Vielzahl von zweiten Rastbefestigungsmitteln eine dritte Anschlagfläche (123) aufweist, die entlang eines Umfangs der Führungsabdeckung (1) angeordnet ist, und jede der Vielzahl von zweiten Rastaussparungen eine vierte Anschlagfläche (243) zum Eingriff mit einer entsprechenden dritten Anschlagfläche (123) aufweist, und wobei die dritten Anschlagflächen (123) konfiguriert sind, um an den entsprechenden vierten Anschlagflächen (243) anzuliegen, um die Umfangsdrehung der Führungsabdeckung (1) in Bezug auf die Führungsbasis (2) zu begrenzen; und/oder
jedes der Vielzahl von zweiten Rastbefestigungsmitteln eine dritte Rastfläche (122) aufweist, die dem zweiten Ende (102) zugewandt ist, und jede der Vielzahl von zweiten Rastaussparungen eine vierte Rastfläche (242) zum Eingreifen in eine entsprechende dritte Rastfläche (122) aufweist, und wobei die dritte Rastfläche (122) konfiguriert ist, um an der vierten Rastfläche (242) anzuliegen, nachdem die Führungsbasis (2) in das erste innere Lumen (100) eingeführt wurde, um die axiale Verschiebung der Führungsabdeckung (1) in Bezug auf die Führungsbasis (2) zu begrenzen.

6. Ladewerkzeug für ein Implantat nach Anspruch 3, wobei die Vielzahl von zweiten Rastbefestigungsmitteln gleichmäßig über den Umfang der Führungsabdeckung (1) verteilt sind, und wobei die Vielzahl von zweiten Rastaussparungen gleichmäßig über den Umfang der Führungsbasis (2) verteilt sind.

7. Ladewerkzeug für ein Implantat nach Anspruch 3, wobei wenn die Einrastpassung zwischen der ersten Raste (17) und der ersten Rastaufnahme (22) gebildet ist, jedes von den zweiten Rastbefestigungsmitteln teilweise in einer entsprechenden zweiten Rastaussparung aufgenommen ist, wodurch die axiale und radiale Verschiebung und die Umfangsdrehung der Führungsabdeckung (1) in Bezug auf die Führungsbasis (2) begrenzt wird.

8. Ladewerkzeug für ein Implantat nach Anspruch 1, wobei das erste innere Lumen (100) eine vom ersten Ende (101) zum zweiten Ende (102) allmählich abnehmende Querschnittsfläche aufweist, und vorzugsweise
das erste innere Lumen (100) einen ersten Abschnitt (13), einen zweiten Abschnitt (16), einen dritten Abschnitt (15) und einen vierten Abschnitt (14) umfasst, die nacheinander von dem ersten Ende (101) zu dem zweiten Ende (102) verbunden sind,
wobei der erste Abschnitt (13) einen konstanten Innendurchmesser entlang der axialen Richtung der Führungsabdeckung (1) aufweist, und wobei ein Endabschnitt des ersten Abschnitts (13) nahe dem ersten Ende (101) eine Vielzahl von Schlitzen (180) aufweist, die entlang einer Umfangsrichtung gebildet sind,
wobei der zweite Abschnitt (16) an einem Ende, das näher am ersten Ende (101) liegt, einen Innendurchmesser aufweist, der größer ist als ein Innendurchmesser des vierten Abschnitts (14) an einem Ende, das näher an dem zweiten Ende (102) ist,
wobei ein Innendurchmesser des dritten Abschnitts (15) an einem Ende davon, das näher an dem ersten Ende (101) ist, und ein Innendurchmesser des dritten Abschnitts (15) an einem Ende davon, das näher an dem zweiten Ende (102) ist, jeweils kleiner ist als der Innendurchmesser des zweiten Abschnitts (16) an dem Ende davon, das näher an dem ersten Ende (101) ist, und größer ist als der Innendurchmesser des vierten Abschnitts (14) an dem Ende davon, das näher an dem zweiten Ende (102) ist.

9. Ladewerkzeug für ein Implantat nach Anspruch 1, wobei die Führungsbasis (2) ein zweites inneres Lumen (200) aufweist, das sich durch sie hindurch entlang der axialen Richtung der Führungsbasis (2) erstreckt, und wobei das zweite innere Lumen (200) eine Querschnittsfläche aufweist, die von dem dritten Ende (201) zu dem vierten Ende (202) allmählich abnimmt, und vorzugsweise
das zweite innere Lumen (200) nacheinander einen fünften Abschnitt (26) und einen sechsten Abschnitt (25) umfasst, die von dem dritten Ende zu dem vierten Ende verbunden sind,
wobei der fünfte Abschnitt (26) an einem Ende, das näher an dem dritten Ende (201) ist, einen Innendurchmesser aufweist, der größer ist als ein Innendurchmesser des fünften Abschnitts (26) an einem Ende, das näher an dem vierten Ende (202) ist, und
wobei der sechste Abschnitt (25) an seinem dem dritten Ende (201) näheren Ende einen Innendurchmesser aufweist, der gleich wie der Innendurchmesser des fünften Abschnitts (26) an seinem dem vierten Ende (202) näheren Ende ist und nicht kleiner als ein Innendurchmesser des sechsten Abschnitts (25) an seinem dem vierten Ende (202) näheren Ende ist.

10. Ladewerkzeug für ein Implantat nach Anspruch 1, wobei ein Außenumfang der Führungsbasis (2) mit Verankerungsgräben (23) mit Einkerbungen versehen ist, und wobei die Einkerbungen der Verankerungsgräben (23) zu dem vierten Ende (202) ausgerichtet sind.

11. Ladewerkzeug für ein Implantat nach Anspruch 1, wobei wenn die Einrastpassung zwischen der zweiten Raste (12) und der zweiten Rastaufnahme (24) gebildet ist, das vierte Ende (202) entlang einer Richtung von dem ersten Ende (101) zu dem zweiten Ende (102) über das zweite Ende (102) hervorsteht.

12. Ladewerkzeug für ein Implantat nach Anspruch 1, wobei sowohl die Führungsabdeckung (1) als auch die Führungsbasis (2) mit Abrutschsicherungsmerkmalen (19) an oder in ihrer äußeren Umfangswand versehen sind, um ein Ergreifen zu erleichtern.

13. Ladewerkzeug für ein Implantat nach einem der Ansprüche 1 bis 12, wobei die Führungsabdeckung (1) aus einem transparenten Material gebildet ist und wobei die Führungsabdeckung (1) mit einem Indikatorring (11) versehen ist, der in Umfangsrichtung um die Führungsabdeckung (1) angeordnet ist, um einen Fortschritt beim Laden eines Implantats anzugeben.

14. Medizinische Vorrichtung, umfassend das Ladewerkzeug für ein Implantat, wie definiert in einem der Ansprüche 1 bis 13, und eine Zuführungsvorrichtung (5), wobei das Ladewerkzeug konfiguriert ist, um mit der Zuführungsvorrichtung (5) zusammenzuwirken, um ein Implantat in die Zuführungsvorrichtung zu laden.

15. Verfahren nach Anspruch 14, wobei das Implantat ein Ventil-Stent (9) ist.

## Revendications

1. Outil de chargement pour un implant, comprenant un couvercle de guidage (1) et une base de guidage (2) couplée de manière détachable au couvercle de guidage (1), dans lequel : le couvercle de guidage (1) a une première extrémité (101) et une deuxième extrémité (102) opposée à la première extrémité (101) le long de sa direction axiale ; la base de guidage (2) a une troisième extrémité (201) et une quatrième extrémité (202) opposée à la troisième extrémité (201) le long de sa direction axiale ; le couvercle de guidage (1) a une première lumière intérieure (100) s'étendant à travers celui-ci ; et la base de guidage (2) est configurée pour être insérée dans la première lumière intérieure (100) depuis la première extrémité (101) vers la deuxième extrémité (102) dans une direction allant de la troisième extrémité (201) vers la quatrième extrémité (202),
dans lequel l'un du couvercle de guidage (1) et de la base de guidage (2) est pourvu d'une première partie d'encliquetage (17) et d'une seconde partie d'encliquetage (12), et l'autre du couvercle de guidage (1) et de la base de guidage (2) est pourvu d'une première base d'encliquetage (22) et d'une seconde base d'encliquetage (24), la première partie d'encliquetage (17) est configurée pour former un ajustement par encliquetage avec la première base d'encliquetage (22) afin de limiter un déplacement radial et axial du couvercle de guidage (1) par rapport à la base de guidage (2), la seconde partie d'encliquetage (12) est configurée pour former un ajustement par encliquetage avec la seconde base d'encliquetage (24) afin de limiter un déplacement radial et axial et une rotation circonférentielle du couvercle de guidage (1) par rapport à la base de guidage (2), et
dans lequel, pendant l'insertion de la base de guidage (2) dans la première lumière intérieure (100), une distance entre la première partie d'encliquetage (17) et la première base d'encliquetage (22) est plus petite qu'une distance entre la seconde partie d'encliquetage (12) et la seconde base d'encliquetage (24).

2. Outil de chargement pour un implant selon la revendication 1, dans lequel les première et seconde parties d'encliquetage (17, 12) sont prévues sur le couvercle de guidage (1), et les première et seconde bases d'encliquetage (22, 24) sont prévues sur la base de guidage (2), dans lequel la première partie d'encliquetage (17) a une première surface d'encliquetage (171) faisant face à la deuxième extrémité (102) et une première surface de mise en prise (172) faisant face à un intérieur du couvercle de guidage (1), dans lequel la première base d'encliquetage (22) a une deuxième surface d'encliquetage (221) faisant face à la troisième extrémité (201) et une deuxième surface de mise en prise (222) faisant face à un extérieur de la base de guidage (2), et dans lequel la première surface de mise en prise (172) est configurée pour venir en butée contre la seconde surface de mise en prise (222) de façon à limiter le déplacement radial du couvercle de guidage (1) par rapport à la base de guidage (2), et la première surface d'encliquetage (171) est configurée pour venir en butée contre la seconde surface d'encliquetage (221) de façon à limiter le déplacement axial du couvercle de guidage (1) par rapport à la base de guidage (2), et ainsi former l'ajustement par encliquetage entre la première partie d'encliquetage (17) et la première base d'encliquetage (22).

3. Outil de chargement pour un implant selon la revendication 2, dans lequel la seconde partie d'encliquetage (12) comprend une pluralité de secondes fixations par encliquetage et la seconde base d'encliquetage (24) comprend une pluralité de seconds évidements d'encliquetage configurés pour une mise en prise avec des secondes fixations par encliquetage respectives, et dans lequel les seconds évidements d'encliquetage sont configurés pour limiter :
le déplacement radial et la rotation circonférentielle du couvercle de guidage (1) par rapport à la base de guidage (2) pendant l'insertion de la base de guidage (2) dans la première lumière intérieure (100) ; et
le déplacement radial et axial et la rotation circonférentielle du couvercle de guidage (1) par rapport à la base de guidage (2) après que la base de guidage (2) a été insérée dans la première lumière intérieure (100).

4. Outil de chargement pour un implant selon la revendication 3, dans lequel chacune de la pluralité de secondes fixations par encliquetage a un premier biseau (121) de préférence pourvu d'une rainure antidérapante, et chacun de la pluralité de seconds évidements d'encliquetage a un second biseau (241) destiné à venir en prise avec un premier biseau (121) correspondant, dans lequel le premier biseau (121) est incliné selon un angle par rapport à la direction axiale du couvercle de guidage (1), dans lequel le second biseau (241) est configuré pour venir en butée contre le premier biseau (121) pendant l'insertion de la base de guidage (2) dans la première lumière intérieure (100).

5. Outil de chargement pour un implant selon la revendication 3, dans lequel chacune de la pluralité de secondes fixations par correspondant a une troisième surface d'arrêt (123) agencée le long d'une circonférence du couvercle de guidage (1) et chacun de la pluralité de seconds évidements d'encliquetage a une quatrième surface d'arrêt (243) destinée à venir en prise avec une troisième surface d'arrêt (123) correspondante, et dans lequel les troisièmes surfaces d'arrêt (123) sont configurées pour venir en butée contre les quatrièmes surfaces d'arrêt (243) correspondantes de manière à limiter la rotation circonférentielle du couvercle de guidage (1) par rapport à la base de guidage (2) ; et/ou
chacune de la pluralité de secondes fixations par encliquetage possède une troisième surface d'encliquetage (122) faisant face à la deuxième extrémité (102) et chacun de la pluralité de seconds évidements d'encliquetage possède une quatrième surface d'encliquetage (242) destinée à venir en prise avec une troisième surface d'encliquetage (122), et dans lequel la troisième surface d'encliquetage (122) est configurée pour venir en butée contre la quatrième surface d'encliquetage (242) après que la base de guidage (2) a été insérée dans la première lumière interne (100) de manière à limiter le déplacement axial du couvercle de guidage (1) par rapport à la base de guidage (2).

6. Outil de chargement pour un implant selon la revendication 3, dans lequel la pluralité de secondes fixations par encliquetage sont distribuées de manière égale autour d'une circonférence du couvercle de guidage (1), et dans lequel la pluralité de seconds évidements d'encliquetage sont distribués de manière égale autour d'une circonférence de la base de guidage (2).

7. Outil de chargement pour un implant selon la revendication 3, dans lequel, lorsque l'ajustement par encliquetage entre la première partie d'encliquetage (17) et la première base d'encliquetage (22) est formé, chacune des secondes fixations par encliquetage est partiellement reçue dans un second évidement d'encliquetage correspondant, limitant ainsi le déplacement axial et radial et la rotation circonférentielle du couvercle de guidage (1) par rapport à la base de guidage (2).

8. Outil de chargement pour un implant selon la revendication 1, dans lequel la première lumière interne (100) a une section transversale qui diminue progressivement de la première extrémité (101) à la deuxième extrémité (102), et de préférence
la première lumière intérieure (100) comprend une première section (13), une deuxième section (16), une troisième section (15) et une quatrième section (14), qui sont jointes séquentiellement de la première extrémité (101) à la deuxième extrémité (102),
dans lequel la première section (13) a un diamètre intérieur constant le long de la direction axiale du couvercle de guidage (1), et dans lequel une partie d'extrémité de la première section (13) proche de la première extrémité (101) a une pluralité de fentes (180) formées le long d'une direction circonférentielle,
dans lequel la deuxième section (16) a un diamètre intérieur à une extrémité de celle-ci plus proche de la première extrémité (101) qui est plus grand qu'un diamètre intérieur de la quatrième section (14) à une extrémité de celle-ci plus proche de la deuxième extrémité (102),
dans lequel chacun d'un diamètre intérieur de la troisième section (15) à une extrémité de celle-ci plus proche de la première extrémité (101) et d'un diamètre intérieur de la troisième section (15) à une extrémité de celle-ci plus proche de la deuxième extrémité (102) est plus petit que le diamètre intérieur de la deuxième section (16) à l'extrémité de celle-ci plus proche de la première extrémité (101) et est plus grand que le diamètre intérieur de la quatrième section (14) à l'extrémité de celle-ci plus proche de la deuxième extrémité (102).

9. Outil de chargement pour un implant selon la revendication 1, dans lequel la base de guidage (2) a une seconde lumière intérieure (200) s'étendant à travers celle-ci le long de la direction axiale de la base de guidage (2), et dans lequel la seconde lumière intérieure (200) a une section transversale diminuant progressivement de la troisième extrémité (201) à la quatrième extrémité (202), et de préférence
la seconde lumière intérieure (200) comprend séquentiellement une cinquième section (26) et une sixième section (25), qui sont jointes de la troisième extrémité à la quatrième extrémité,
dans lequel la cinquième section (26) a un diamètre intérieur à une extrémité de celle-ci plus proche de la troisième extrémité (201) qui est plus grand qu'un diamètre intérieur de la cinquième section (26) à une extrémité de celle-ci plus proche de la quatrième extrémité (202), et
dans lequel la sixième section (25) a un diamètre intérieur à une extrémité de celle-ci plus proche de la troisième extrémité (201) qui est égal au diamètre intérieur de la cinquième section (26) à l'extrémité de celle-ci plus proche de la quatrième extrémité (202), et qui n'est pas inférieur à un diamètre intérieur de la sixième section (25) à une extrémité de celle-ci plus proche de la quatrième extrémité (202).

10. Outil de chargement pour un implant selon la revendication 1, dans lequel une circonférence extérieure de la base de guidage (2) est pourvue de tranchées d'ancrage (23) avec des encoches, et dans lequel les encoches des tranchées d'ancrage (23) sont orientées vers la quatrième extrémité (202).

11. Outil de chargement pour un implant selon la revendication 1, dans lequel, lorsque l'ajustement par encliquetage entre la seconde partie d'encliquetage (12) et la seconde base d'encliquetage (24) est formé, la quatrième extrémité (202) fait saillie au-delà de la deuxième extrémité (102) le long d'une direction allant de la première extrémité (101) vers la deuxième extrémité (102).

12. Outil de chargement pour un implant selon la revendication 1, dans lequel chacun du couvercle de guidage (1) et de la base de guidage (2) est pourvu d'éléments antidérapants (19) sur ou dans sa paroi circonférentielle extérieure pour faciliter la préhension.

13. Outil de chargement d'un implant selon l'une quelconque des revendications 1 à 12, dans lequel le couvercle de guidage (1) est formé d'un matériau transparent, et dans lequel le couvercle de guidage (1) est muni d'un anneau indicateur (11) disposé circonférentiellement autour du couvercle de guidage (1) pour indiquer une progression du chargement d'un implant.

14. Dispositif médical comprenant l'outil de chargement pour un implant selon l'une quelconque des revendications 1 à 13 et un dispositif de délivrance (5), dans lequel l'outil de chargement est configuré pour coopérer avec le dispositif de délivrance (5) pour charger un implant dans le dispositif de délivrance.

15. Dispositif médical selon la revendication 14, dans lequel l'implant est un stent valvulaire (9).
